# EUROPÄISCHE PATENTSCHRIFT

(11) **EP 2 408 403 B1**
(45) Veröffentlichungstag und Bekanntmachung des Hinweises auf die Patenterteilung: **29.05.2019**
(21) Anmeldenummer: 10710798.9
(22) Anmeldetag: 17.03.2010
(51) Int. Cl.: B01F 7/00, A61B 17/88, B01F 15/02, A61B 90/00

(54) **VAKUUM-MISCHVORRICHTUNG FÜR KNOCHENZEMENT SOWIE VERFAHREN ZUM MISCHEN VON KNOCHENZEMENT IN DER VORRICHTUNG**
VACUUM-MIXING DEVICE FOR BONE CEMENT AND PROCESS FOR MIXING BONE CEMENT IN SAID DEVICE
DISPOSITIF DESTINÉ À MÉLANGER DU CIMENT OSSEUX SOUS VIDE ET PROCÉDÉ DE MÉLANGER DU CIMENT OSSEUX AVEC CE DISPOSITIF

(30) Priorität: 17.03.2009 DE 102009013211
(43) Veröffentlichungstag der Anmeldung: 25.01.2012
(73) Patentinhaber: Stryker European Holdings I, LLC, Kalamazoo, MI 49002 (US)
(72) Erfinder: BIELENSTEIN, Oliver, 14193 Berlin (DE); SATTIG, Christoph, 64807 Dieburg (DE); DEUSSER, Stefan, 63791 Karlstein (DE); STIRNAL, Volker, 64807 Dieburg (DE)
(74) Vertreter: Regimbeau
(86) Internationale Anmeldenummer: PCT/EP2010/001665
(87) Internationale Veröffentlichungsnummer: WO 2010/105807

(56) Entgegenhaltungen:
- EP-A1- 1 886 648
- EP-A2- 0 493 363
- EP-A2- 1 020 167
- WO-A1-00/35506
- WO-A1-00/43116
- WO-A1-96/07472
- WO-A1-03/031042
- US-A- 5 443 182
- US-A1- 2003 155 381
- US-B1- 6 286 670

## Beschreibung

### Gebiet der Erfindung

Die Erfindung betrifft eine Vakuum-Mischvorrichtung für Knochenzement sowie Verfahren zum Anmischen von Knochenzement.

### Hintergrund der Erfindung

Mischvorrichtungen für Knochenzement sind bekannt.

Eine derartige gattungsbildende Mischvorrichtung zeigt beispielsweise die deutsche Patentanmeldung DE 10 2007 041 666 A1 der Anmelderin. Beim Anmischen von Knochenzement wird üblicherweise ein Pulver, insbesondere ein PMMA-Pulver, mit einem flüssigen Monomer vermischt. Zur Vermeidung von Blasenbildungen und/oder um zu vermeiden, dass schädliche gasförmige Substanzen, die beim Anmischen entstehen, in die Umwelt gelangen, sind sogenannte Vakuum-Mischsysteme bekannt. Es handelt sich dabei um Systeme, bei welchen der Mischbehälter einen Anschluss für einen Unterdruckschlauch aufweist, über den er während des Mischvorgangs unter Vakuum gesetzt wird.

Bekannte herkömmliche Systeme erfordern oft eine relativ komplizierte Bedienung, bei der der Benutzer mehrere Schritte machen muss, die mit Fehlerquellen verbunden sind.

Insbesondere ist wichtig, dass das vorgegebene Mischungsverhältnis von Monomer und Pulver genau eingehalten wird.

Dazu sind beispielsweise Systeme bekannt, bei welchen das Monomer aufgrund des im Mischbehälter anliegenden Vakuums in den Mischbehälter hinein gesaugt wird. Derartige herkömmliche Systeme verfügen in der Regel über ein Ventil, durch welches Luft in den Monomer-Behälter einströmen kann, so dass die vorhandene Flüssigkeit vollständig aus dem Monomer-Behälter austritt. Ein solches System ist beispielsweise in der europäischen Patentschrift EP 0 725 647 B1 beschrieben.

Nachteilig an einem solchen System ist, insbesondere wenn dieses in einer nicht sterilen Umgebung betrieben wird, dass der Knochenzement mit Keimen aus der Raumluft kontaminiert werden kann.

WO 9607472 A1 beschreibt Verfahren und Vorrichtungen zum Zubereiten und Abgeben von Knochenzement durch Kombinieren eines pulverförmigen Polymer-und einer flüssigen Monomerkomponente. Die pulverförmige Polymer-Komponente ist in einer evakuierten Mischkammer gespeichert. Die flüssige Monomerkomponente wird in die Mischkammer eingeführt und mit dem Pulver vermischt.Hierbei wird das flüssige Monomer in der vakuumverpackten Pulverkammer, unter der Wirkung des Vakuums in einer zeitlichen Abfolge unter Verwendung einer Vielzahl von Monomer-Verteilungskanäle (105 , 299, 208) verteilt.

US 6,286,670 B1 beschreibt einen Behälter für zwei Komponenten, die miteinander vermischt werden sollen. Der Behälter weist einen Teiler und einen Sperrmechanismus auf, wobei der Teiler eine erste Kammer mit einer flüssigen Komponente von einer zweiten Kammer zur Aufnahme des Pulvers trennt. Eine Vakuumquelle ist in Fluidverbindung mit der zweiten Kammer platziert, sodass die erste Komponente durch die zweite Komponente gezogen werden kann, um eine gründliches Mischen sicherzustellen.

WO 00/43116 A1 beschreibt einen Knochenzement-Mischer mit einem Patronengehäuse zur Aufnahme einer offenen Knochenzement-Patrone mit Knochenzement-Pulver, mit einem Rührer in der Patrone und einer Abdeckung über dem Gehäuse, welches eine Vakuumkammer definiert. Die Kammer kann evakuiert werden, wodurch eine Monomer-Flüssigkeit in das Knochenzement-Pulver derart strömt, dass sie das Knochenzement-Pulver durchdringt und die Monomer-Flüssigkeit im Wesentlichen gleichmäßig entlang der Höhe des Knochenzement-Pulvers verteilt wird. Das Pulver und die Flüssigkeit werden anschließend mechanisch vermischt, um Knochenzement zu bilden.

WO 0035506 A1 beschreibt ein Verfahren zur Herstellung von Knochenzement aus einem polymeren Pulver und einer flüssigen Komponente. Die Partikel der pulverförmigen Komponente können in einem Pulverbehälter mit einer Einlaßöffnung und einer Auslaßöffnung verpackt sein, und die flüssige Komponente kann in einem Flüssigkeitsbehälter aufbewahrt sein. Der Flüssigkeitsbehälter kann mit der Einlassöffnung verbunden sein und eine Vakuumquelle mit der Auslaßöffnung. Der Zwischenraum zwischen den Partikeln der pulverförmigen Komponente wird von der flüssigen Komponente durchströmt, wobei die flüssige Komponente durch die Einwirkung der Vakuumquelle von der Einlaßöffnung in Richtung der Auslaßöffnung fließt.

WO 03/031042 A1 beschreibt eine Flüssigkeitstransfer-Anordnung, in der aus zwei Komponenten gebildeter Knochenzement getrennt voneinander gehalten werden können, bis tatsächlich ein Vermischen derselben zur Verwendung erwünscht ist. Eine flüssige Monomerkomponente kann aus einer abgedichteten Einheit direkt in einen Mischbehälter fließen, in einer gegenüber dem Umfeld abgeschlossenen Weise und ohne Bruch des Behälters für die flüssige Monomerkomponente.

Andere Beispiele von Vakuum-Mischvorrichtungen für Knochenzement sind in US 2003/155381 und in WO 03/031042 beschrieben.

### Aufgabe der Erfindung

Der Erfindung liegt demgegenüber die Aufgabe zugrunde, die genannten Nachteile des Standes der Technik zumindest zu reduzieren.

Es ist insbesondere eine Aufgabe der Erfindung, ein Vakuum-Mischsystem für Knochenzement bereitstellen zu können, welches eine einfache und sichere Handhabbarkeit ermöglicht.

### Zusammenfassung der Erfindung

Die Aufgabe der Erfindung wird bereits durch eine Vakuum-Mischvorrichtung sowie durch ein Verfahren zum Anmischen von Knochenzement nach einem der unabhängigen Ansprüche gelöst.

Bevorzugte Ausführungsformen und Weiterbildungen der Erfindung sind den jeweiligen Unteransprüchen zu entnehmen.

Die Erfindung betrifft zum einen eine Vakuum-Mischvorrichtung für Knochenzement, welche einen Mischraum umfasst, in welchem ein Monomer mit dem Pulver vermischt werden kann. Um Blasenbildung und das Austreten von schädlichen Gasen zu verhindern, ist der Mischraum an eine Vakuumquelle, insbesondere an eine Vakuumpumpe anschließbar.

Weiter umfasst die Vakuum-Mischvorrichtung einen Monomerbehälter, welcher mit dem Mischraum verbindbar ist. Vorzugsweise ist das Monomer bereits bei Auslieferung der Vakuum-Mischvorrichtung in dem Monomerbehälter enthalten, muss also nicht aus einem getrennten Behältnis entnommen und eingefüllt werden. Unter "verbindbar" wird verstanden, dass die Vakuum-Mischvorrichtung Mittel aufweist, um eine Passage auszubilden, in welcher das Monomer vom Monomerbehälter in den mit dem Monomerbehälter verbundenen Mischbehälter fließt.

Gemäß der Erfindung ist der geschlossene Monomerbehälter teilweise mit einem Gas gefüllt, dessen Volumen so bemessen ist, dass bei anliegendem Vakuum das Monomer von dem Gas verdrängt wird und so in den Mischbehälter fließt.

Vorzugsweise steht der Monomerbehälter nicht unter Überdruck, sondern hat beispielsweise im Wesentlichen Atmosphärendruck. So ist eine einfachere Ausgestaltung des Behältnisses möglich und es wird ein unkontrolliertes Austreten des Monomers bei Beschädigungen verhindert.

Sofern der Monomerbehälter beispielsweise unter Atmosphärendruck steht und eine Vakuumquelle angelegt wird, die in dem Mischbehälter ein Vakuum von etwa 0,5 bar erzeugt, muss folglich das Gasvolumen im Monomerbehälter mindestens die Hälfte des Volumens einnehmen.

Durch die Erfindung wird eine vollständig abgeschlossene Ausbildung der Vakuummischvorrichtung ermöglicht, so dass beim Mischen nicht die Gefahr besteht, dass durch angesaugte Luft Keime den Knochenzement verunreinigen.

Als Gas kann im einfachsten Falle Luft verwendet werden aber auch andere Gase, insbesondere solche, die eine konservierende Schutzatmosphäre ausbilden, sind denkbar. Beispielsweise kann auch Stickstoff oder ein Edelgas verwendet werden.

Bei einer bevorzugten Ausführungsform der Erfindung ist das Gasvolumen derart groß, dass das Monomer bei anliegendem Vakuum bis zu einem vorgegebenen Füllstand oder vollständig in den Mischbehälter fließt.

Eine vorgegebene Restfüllmenge kann beispielsweise durch die Höhe der beim Mischen in den Monomerbehälter hineinragenden Kanüle bestimmt werden. Je nach Ausgestaltung ist auch eine vollständige Entleerung des Monomerbehälters möglich.

Bei dieser Ausführungsform der Erfindung ist eine exakte Eichung der abzugebenden Flüssigkeitsmenge möglich, da die Entleerung des Monomerbehälters komplett automatisch erfolgt und der Benutzer keinen Einfluss auf die im Monomerbehälter verbleibende Restmenge hat, wie es beispielsweise beim Einfüllen mit einer Spritze der Fall ist.

Gemäß der Erfindung ist der Monomerbehälter zumindest zu 20, vorzugsweise zu 40 % seines Volumens mit dem Gas gefüllt.

Bei einer Weiterbildung der Erfindung weist der Monomerbehälter eine Membran auf und die Vakuum-Mischvorrichtung umfasst eine Einstechkanüle, über welche die Membran eingestochen werden kann und so das Monomer über die Kanüle in den Mischbehälter fließt.

Diese Ausführungsform der Erfindung ermöglicht insbesondere die Verwendung handelsüblicher Monomerfläschchen aus Glas, welche mit einer Membran versehen sind. In dem Monomerfläschchen ist das Monomer so sicher und steril aufbewahrt.

Weiter betrifft die Erfindung eine Vakuum-Mischvorrichtung, welche einen Mischbehälter umfasst, der an eine Vakuumquelle anschließbar ist. Die Vakuum-Mischvorrichtung umfasst einen Monomerbehälter, welcher mit dem Mischbehälter verbindbar ist.

Um eine besonders einfache Ausgestaltung der Vorrichtung zu ermöglichen, ist der Monomerbehälter an einem Griff angeordnet, mit dem zugleich ein Mischpaddel im Mischraum betätigbar ist, mit welchem das Monomer mit dem Pulver manuell durch Aufwärts- und Abwärtsbewegen des Mischpaddels vermischt werden kann.

Zum Bewegen des Mischpaddels ist eine Stange mit dem Griff verbunden.

Gemäß der Erfindung ist die Stange in einer Platte geführt, welche nach dem Anmischen des Knochenzements als Kolben zum Austreiben des Knochenzements verwendebar ist.

Vorzugsweise umfasst die Stange einen Kanal, welcher genutzt wird, um den Mischbehälter mit dem Monomerbehälter zu verbinden, also um das Monomer in den Mischbehälter fließen zu lassen.

Die Erfindung ermöglicht eine besonders einfach handhabbare Ausgestaltung einer Vakuum-Mischvorrichtung, bei welcher, wie es bei einer bevorzugten Ausführungsform der Erfindung vorgesehen ist, nach Beendigung des Mischvorgangs der Griff mit dem Monomerbehälter abgebrochen werden kann und der Mischbehälter als eine Art Kartusche in eine Dosierpistole eingesetzt wird. Dabei dient die Platte, die zunächst als Führung der Stange dient, als Kolben, um den Knochenzement im Mischbehälter heraus zu drücken.

Gemäß der Erfindung umfasst der Monomerbehälter ein in einer Aufnahmeeinrichtung angeordnetes Monomerfläschchen. Dies ermöglicht unter anderem die Verwendung handelsüblicher Monomerfläschchen und der restliche Monomerbehälter braucht, wie es bei einer bevorzugten Ausführungsform der Erfindung vorgesehen ist, nicht vakuumdicht ausgebildet zu sein, da beispielsweise eine am Monomerfläschchen vorhandene Membran als Dichtelement genutzt werden kann.

Bei einer besonderen Ausführungsform der Erfindung ist die Aufnahmeeinrichtung zusammenschiebbar ausgebildet. So kann beispielsweise ein in der Aufnahmeeinrichtung gehaltenes Monomerfläschchen auf eine Kanüle geschoben werden.

Weiter betrifft die Erfindung eine Vakuum-Mischvorrichtung mit einem Mischraum und einem Monomerbehälter, welcher mit dem Mischraum verbindbar ist.

Gemäß der Erfindung rastet der Monomerbehälter in dem verbundenen Zustand ein und ist vorzugsweise in diesem eingerasteten Zustand verriegelt.

So wird erreicht, dass vom Benutzer die einmal geschaffene Verbindung zwischen Monomerbehälter und Mischbehälter nicht mehr unterbrochen werden kann. Das Monomer tritt daher vollständig in den Mischbehälter ein und es besteht nicht die Gefahr, dass der Benutzer aufgrund unsachgemäßer Anwendung den Monomerbehälter abtrennt, bevor dieser entleert ist.

Gemäß der Erfindung umfasst die Vakuum-Mischvorrichtung einen Sicherheitsriegel, bei dessen Entfernung der Monomerbehälter zur Bewegung in den verbundenen Zustand freigegeben wird. Über einen derartigen Sicherheitsbehälter wird ein unbeabsichtigtes Betätigen der Vorrichtung vermieden.

Vorzugsweise weist die Vakuum-Mischvorrichtung Mittel auf, um ein Wiedereinsetzen des entfernten Sicherheitsriegels zu blockieren. Der einmal gezogene Sicherheitsriegel dient so zugleich als Originalitätssicherung und es ist auf den ersten Blick zu erkennen, ob die Vakuum-Mischvorrichtung bereits benutzt wurde und damit verbraucht ist.

Weiter betrifft die Erfindung ein Verfahren zum Anmischen von Knochenzement. Dabei wird ein Monomer in einem Mischbehälter mit einem Pulver gemischt und zumindest während des Mischens ein Vakuum an den Mischbehälter angelegt.

Gemäß der Erfindung wird das Monomer bei anliegendem Vakuum durch ein aufgrund des Vakuums expandierendes Gas aus dem Monomerbehälter in den Mischbehälter gedrückt.

Das Verfahren ermöglicht eine vollständig geschlossene Ausbildung des Mischsystems.

Gemäß der Erfindung sind daher während des Anmischens Mischbecher und Monomerbehälter von dem Eindringen von Außenluft abgeschnitten.

Bei einer Ausführungsform der Erfindung wird der Monomerbehälter mittels einer Kanüle eingestochen und das Monomer über die Kanüle in den Mischbehälter geleitet.

In dieser verbundenen Stellung verrastet der Monomerbehälter derart, dass das Einlaufen des Monomers nicht mehr unterbrochen werden kann.

Bei einer Weiterbildung der Erfindung wird nach dem Anmischen des Knochenzements der Monomerbehälter vom Mischbehälter abgetrennt und der Mischbehälter als Kartusche für eine Dosierpistole verwendet.

### Kurzbeschreibung der Zeichnungen

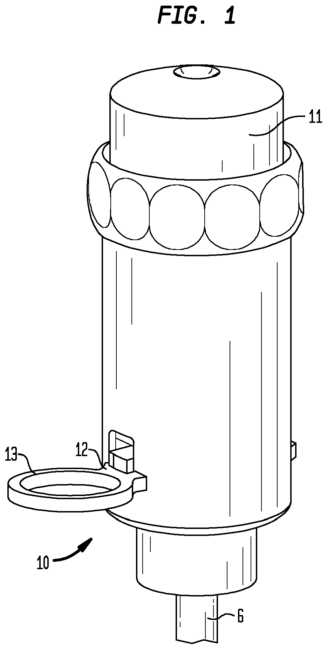

Die Erfindung soll im Folgenden Bezug nehmend auf die Zeichnungen Fig. 1 bis Fig. 6, welche schematisch dargestellte Ausführungsformen der Erfindung zeigen, näher erläutert werden.

Fig. 1 bis Fig. 4 zeigen ein schematisch dargestelltes Ausführungsbeispiel eines Griffes mit einem Monomerbehälter für eine erfindungsgemäße Vakuum-Mischvorrichtung.

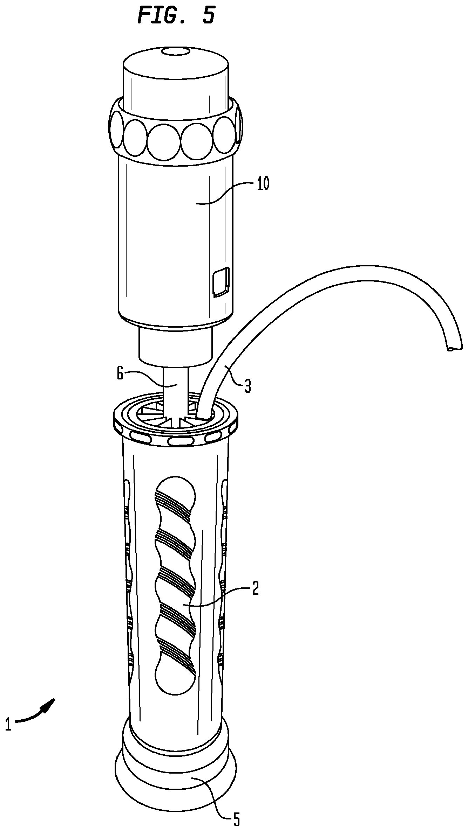

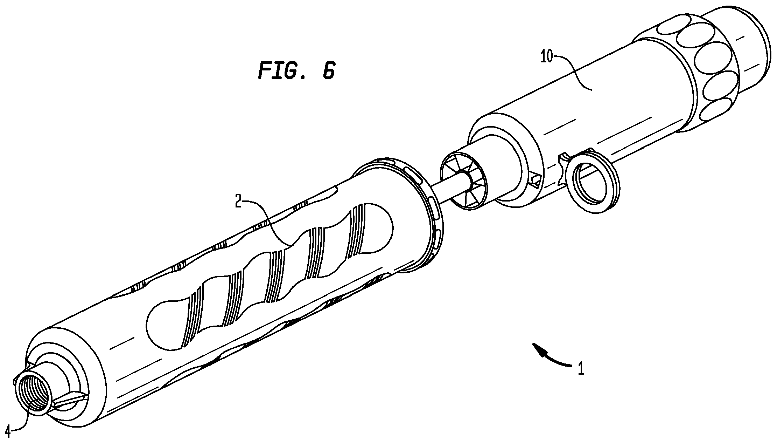

Bezug nehmend auf Fig. 5 und 6 soll die gesamte Vakuum-Mischvorrichtung näher erläutert werden.

### Detaillierte Beschreibung der Zeichnungen

In Fig. 1 ist schematisch ein als Griff für ein Mischpaddel (nicht dargestellt) ausgebildeter Monomerbehälter 10 dargestellt. Der Monomerbehälter 10 hat eine im Wesentlichen zylindrische Form und umfasst einen Betätigungsknopf 11, um das Monomer (nicht dargestellt) freizugeben.

Um den Betätigungsknopf 11 drücken zu können, ist ein Sicherheitsriegel 12 mit einem Griff 13, der ein Fingergriffloch bildet, vorgesehen, welcher zuvor entfernt werden muss.

Nach Entfernung des Sicherheitsriegels 12 kann der Betätigungsknopf 11 gedrückt werden und das Monomer fließt bei Vakuum im Mischbehälter über einen in der Stange 6 eingelassenen Kanal (nicht dargestellt) in den Mischbehälter (nicht dargestellt).

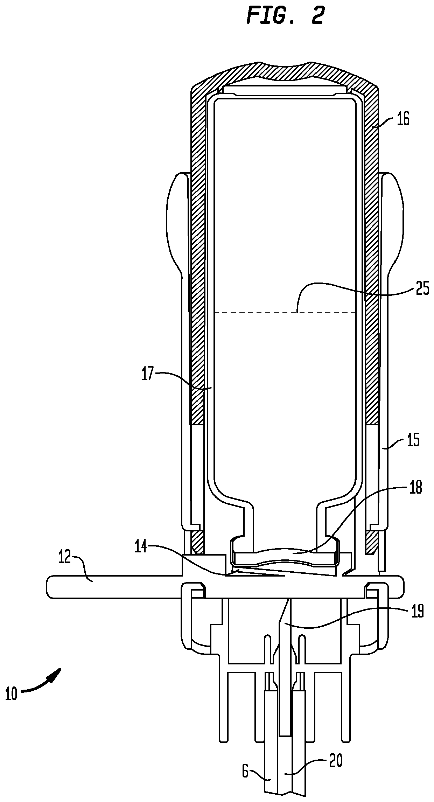

Fig. 2 zeigt eine Schnittansicht des in Fig. 1 dargestellten Monomerbehälters 10 im Ausgangszustand, das heißt vor der Verwendung.

Der Monomerbehälter 10 umfasst ein Gehäuseunterteil 15 und ein Gehäuseoberteil 16, welches gleichzeitig als Betätigungsknopf 11 ausgebildet ist.

Die Gehäuseteile 15, 16 dienen als Aufnahme für ein handelsübliches Monomerfläschchen 17 aus Glas, welches mit einer Membran 18 versiegelt ist.

Das Monomerfläschchen 17 ist in diesem Ausführungsbeispiel etwa zur Hälfte mit dem Monomer gefüllt. Der Füllstand wird durch die Linie 25 vorgegeben.

Weiter umfasst der Monomerbehälter 10 eine Kanüle 19, welche mit einem in der Stange 6 eingelassenen Kanal 20 verbunden ist.

Die Kanüle 19 und die Membran 18 des Monomerfläschchens 17 werden durch den Sicherheitsriegel 12 derart auseinander gehalten, dass eine versehentliche Betätigung nicht möglich ist.

Der Sicherheitsriegel 12 umfasst eine federnde Zunge 14, um Fertigungstoleranzen des Monomerfläschchens 17 auszugleichen.

Bereits nach Herausziehen des Sicherheitsriegels 12 rutscht das Monomerfläschchen 17 in eine Position, in der der Sicherheitsriegel 12 nicht erneut eingesetzt werden kann.

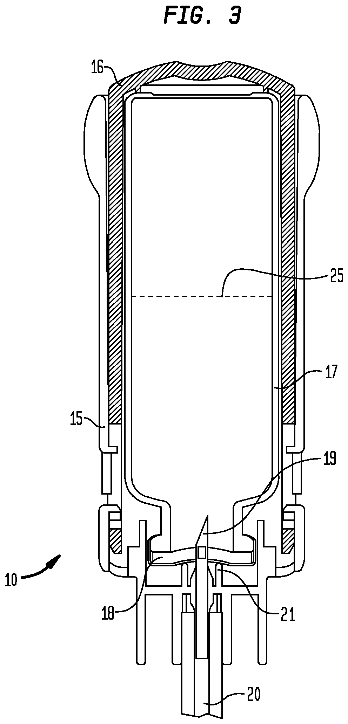

In Fig. 3 ist der Monomerbehälter 10 nach einer Betätigung in der Schnittansicht dargestellt.

Mit Drücken des Betätigungsknopfes werden Gehäuseoberteil 16 und Gehäuseunterteil 15 ineinander geschoben, wodurch das Monomerfläschchen 17 auf die Kanüle 19 aufgeschoben wird, welche die Membran 18 durchsticht, so dass das Monomer über den Kanal 20 in den Mischbehälter (nicht dargestellt) eintreten kann.

Aufgrund des oberhalb des Füllstandes 25 vorhandenen Luftvolumens und des im Mischbehälter herrschenden Vakuum entleert sich das Monomer bis auf einen geringen Restfüllstand, der durch die Position der Kanüle 19 vorgegeben wird.

Um eine exakte Position der Kanüle 19 zum Monomerfläschchen 17 zu definieren, ist ein Ring 21 im unteren Gehäuseteil vorgesehen, auf welchem das Monomerfläschchen 17 im betätigten Zustand aufsitzt.

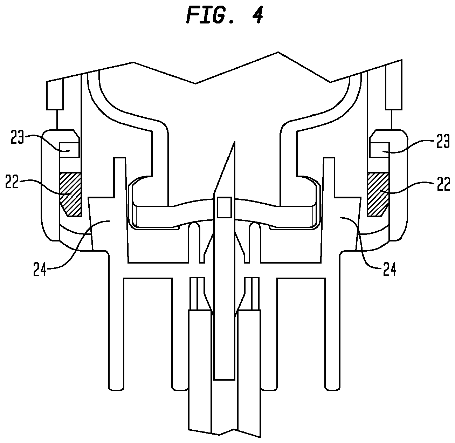

Fig. 4 zeigt eine Detaildarstellung des Kopfes des in Fig. 3 dargestellten Monomerbehälters. In Bezug auf diese Figur soll die Verrastung des Monomerbehälters näher erläutert werden.

Der obere Gehäuseteil umfasst Rasthaken 22, welche im betätigten Zustand unter Rastnasen 23 des unteren Gehäuseteils greifen. Dazu sind die Rasthaken 22 angeschrägt. Ein Wiederauseinanderziehen ist nicht möglich.

Um die Manipulationssicherheit weiter zu erhöhen, sind am unteren Gehäuseteil Rippen 24 vorgesehen, über die weitgehend verhindert wird, dass mit einem Werkzeug von unten die Rasthaken nach innen geschoben werden können und so das Monomerfläschchen in seine Ursprungsposition gebracht werden kann.

Fig. 5 zeigt schematisch dargestellt die komplette Vakuum-Mischvorrichtung.

Der Monomerbehälter 10 ist als Griff ausgebildet, welcher über eine Stange 6 mit einem Mischpaddel (nicht dargestellt) verbunden ist.

Der Mischbehälter 2 ist vorzugsweise bereits mit dem Pulver vorgefüllt.

Damit das Pulver nicht in die Stange 6 eintritt, ist am unteren Ende der Stange ein Filter, insbesondere eine Filterscheibe vorgesehen (nicht dargestellt).

Bei Verwendung der Vakuum-Mischvorrichtung 1 wird am Mischbehälter 2 an einem Vakuumanschluss 3 eine Vakuumpumpe angeschlossen. Ein in den Vakuumanschluss eingelassenes Vlies (nicht dargestellt) verhindert dabei das Austreten von Knochenzement.

Am Boden des Mischbehälters 2 befindet sich die Entnahmeöffnung, welche anfänglich noch mit einem Standfuß 5 verschraubt ist.

Der Benutzer (nicht dargestellt) schließt nunmehr zunächst den Mischbehälter 2 am Vakuum an. Danach zieht er den Sicherheitsriegel und drückt den Betätigungsknopf 11 des Monomerbehälters.

Aufgrund des Vakuums wird das Monomer (nicht dargestellt) von dem im Monomerbehälter 10 eingeschlossenen Gas verdrängt und fließt über die Stange 6 in den Mischbehälter 2. Die Stange 6 umfasst dazu seitliche Öffnungen über den Mischpaddeln (nicht dargestellt).

Der Benutzer verwendet sodann den Monomerbehälter 10 als Griff, um die Mischpaddel (nicht dargestellt) im Mischbehälter 2 zu bewegen und so den Knochenzement anzumischen.

Im Anschluß zieht der Benutzer das Mischpaddel ganz nach oben und bricht die Stange 6 komplett mit dem Monomerbehälter 10 an einer Sollbruchstelle ab.

Des Weiteren schraubt der Benutzer das Fußteil 5 ab, so dass die Entnahmeöffnung freigegeben wird.

Der Mischbehälter 2 kann nunmehr als Kartusche für eine Dosierpistole (nicht dargestellt) verwendet werden.

Dabei dient der Deckel 7 des Mischbehälters 2, in welchem zugleich die Stange 6 geführt wird, als Kolben. Der Deckel 7 ist hierzu derart mit dem restlichen Mischbehälter 2 verbunden, dass er sich ab einer bestimmten Kraft ablöst und sodann vorgeschoben werden kann. Beispielsweise kann der Deckel in einer Nut des zylinderförmigen Gehäuses des Mischbehälters 2 eingepresst sein.

Fig. 6 zeigt eine weitere perspektivische Ansicht einer Vakuum-Mischvorrichtung 1. In dieser Ansicht ist bereits der Standfuß (5 in Fig. 5) abgeschraubt, so dass die Entnahmeöffnung 4 zu sehen ist.

Die Erfindung ermöglicht eine besonders einfache und sichere Handhabung von Knochenzement.

Es versteht sich, dass die Erfindung nicht auf eine Kombination vorstehend beschriebener Merkmale beschränkt ist, sondern dass der Fachmann sämtliche Merkmale, soweit es sinnvoll ist, kombinieren wird.

### Bezugszeichenliste

- 1: Vakuum-Mischvorrichtung
- 2: Mischbehälter
- 3: Vakuumanschluss
- 4: Entnahmeöffnung
- 5: Standfuß
- 6: Stange
- 7: Deckel
- 10: Monomerbehälter
- 11: Betätigungsknopf
- 12: Sicherheitsriegel
- 13: Griff
- 14: Zunge
- 15: Gehäuseunterteil
- 16: Gehäuseoberteil
- 17: Monomerfläschchen
- 18: Membran
- 19: Kanüle
- 20: Kanal
- 21: Ring
- 22: Rasthaken
- 23: Rastnase
- 24: Rippe
- 25: Füllstand

## Patentansprüche

1. Vakuum-Mischvorrichtung (1) für Knochenzement, mit einem Mischbehälter (2), welcher an eine Vakuumquelle anschliessbar ist, und einem Monomerbehälter (10), welcher ein Fläschchen (17) mit einem Monomer aufweist, wobei der Monomerbehälter (10) mit dem Mischbehälter verbindbar ist, wobei das Fläschchen (17) zumindest zu 20 % seines Volumens mit einem Gas gefüllt ist, wenn kein Vakuum in dem Mischbehälter anliegt, so dass das Monomer aus dem Monomerbehälter in den Mischbehälter fliesst, wenn ein Vakuum am Mischbehälter (2) anliegt, und wobei die Vakuum-Mischvorrichtung (1) geschlossen ist, sodass keine Umgebungsluft in das Fläschchen (17) oder den Mischbehälter (2) eindringen kann,
**dadurch gekennzeichnet, dass** die Vakuum-Mischvorrichtung einen Sicherheitsriegel (12) umfasst, bei dessen Entfernung der Monomerbehälter (10) zur Bewegung in einen mit dem Mischbehälter (2) verbundenen Zustand freigegeben wird.

2. Vakuum-Mischvorrichtung (1) nach Anspruch 1, wobei das Gas Luft ist.

3. Vakuum-Mischvorrichtung (1) nach einem der vorstehenden Ansprüche, wobei das Fläschchen zumindest zu 40 % seines Volumens mit dem Gas gefüllt ist.

4. Vakuum-Mischvorrichtung (1) nach einem der vorstehenden Ansprüche, wobei das Fläschchen (17) eine Membran (18) aufweist und die Vakuum-Mischvorrichtung eine Einstechkanüle (19) zum Durchstechen der Membran umfasst, über die das Monomer in den Mischbehälter (2) fliesst.

5. Vakuum-Mischvorrichtung (1) nach dem vorstehenden Anspruch, wobei der Monomerbehälter (10) verriegelbar ist, wenn die Membran des Fläschchens von der Einstechkanüle durchstochen ist.

6. Vakuum-Mischvorrichtung (1) nach dem vorstehenden Anspruch, wobei der Monomerbehälter (10) einen Gehäuseoberteil (15) und einen Gehäuseunterteil (16) aufweist, wobei der Gehäuseoberteil (15) und der Gehäuseunterteil (16) das Fläschchen (17) halten und die Teile derart ineinander schiebbar sind, dass das Fläschchen (17) auf die Kanüle (19) geschoben wird.

7. Vakuum-Mischvorrichtung (1) nach dem vorstehenden Anspruch, wobei das Gehäuseoberteil (15) Rasthaken (22) aufweist und das Gehäuseunterteil Rastnasen (23) aufweist, wobei die Rasthaken bei einem Ineinanderschieben der Gehäuseteile in Eingriff mit den Rastnasen bringbar sind, sodass das Gehäuseoberteil (15) und das Gehäuseunterteil (16) nicht in ihre Ursprungsposition vor dem Ineinanderschieben der beiden Gehäuseteile zurück gebracht werden können.

8. Vakuum-Mischvorrichtung (1) nach einem der Ansprüche 5 bis 7, wobei der Mischbehälter (2) eine vorbestimmte Füllmenge aufweist, welche durch eine Höhe der Kanüle (19) bestimmt ist, die in den Monomerbehälter (10) vorsteht.

9. Vakuum-Mischvorrichtung (1) nach einem der vorstehenden Ansprüche, wobei der Monomerbehälter (10) in einem Griff (13) angeordnet ist, mit dem über eine Stange (6) ein Mischpaddel im Mischbehälter betätigbar ist, wobei die Stange (6) in einer Platte geführt ist, welche nach dem Anmischen als Kolben zum Austreiben des Knochenzements dient.

10. Vakuum-Mischvorrichtung (1) nach dem vorstehenden Anspruch, wobei der Monomerbehälter (10) über einen Kanal (20) im Griff (13) mit dem Mischbehälter verbindbar ist.

11. Vakuum-Mischvorrichtung (1) nach dem vorstehenden Anspruch, wobei der Kanal (20) in einer Stange (6) zum Antrieb des Mischpaddels angeordnet ist.

12. Vakuum-Mischvorrichtung (1) nach dem vorstehenden Anspruch, wobei die Stange (6) eine Sollbruchstelle aufweist.

13. Vakuum-Mischvorrichtung (1) nach einem der vorstehenden Ansprüche, wobei, nach Entfernung des Sicherheitsriegels der Monomerbehälter (10) derart freigegeben wird, sodass das Fläschchen auf die Kanüle geschoben werden kann.

14. Vakuum-Mischvorrichtung (1) nach dem vorstehenden Anspruch, wobei die Vakuum-Mischvorrichtung Mittel aufweist, um ein Wiedereinsetzen des entfernten Sicherheitsriegels (12) zu blockieren.

15. Verfahren zum Anmischen von Knochenzement umfassend:
- Mischen von einem Monomer aus einem Fläschchen in einem Mischbehälter mit einem Pulver, wobei das Fläschchen mit dem Mischbehälter verbindbar ist, wenn ein Vakuum an dem Mischbehälter anliegt;
- Entfernen eines Sicherheitsriegels, um das Fläschchen zur Bewegung in einen mit dem Mischbehälter (2) verbundenen Zustand freizugeben; und
- Verbinden des Fläschchens mit dem Mischbehälter, wobei das Monomer bei anliegendem Vakuum durch ein aufgrund des Vakuums expandierendes Gas aus dem Fläschchen in den Mischbehälter gedrückt wird, wobei zumindest 20% des Volumens des Fläschchens mit dem Gas gefüllt ist, wenn kein Vakuum in dem Mischbehälter anliegt, und wobei die Vakuum-Mischvorrichtung derart geschlossen ist, dass keine Umgebungsluft in das Fläschchen oder den Monomerbehälter eindringen kann.

16. Verfahren zum Anmischen von Knochenzement nach dem vorstehenden Anspruch, weiter umfassend: Einstechen des Fläschchens mit einer Kanüle, damit das Monomer in den Mischbehälter fliesst.

17. Verfahren zum Anmischen von Knochenzement nach einem der vorstehenden Ansprüche 15-16, wobei der Monomerbehälter in einer mit der Kanüle verbundenen Stellung verrastet wird, nachdem die Kanüle das Fläschchen eingestochen hat.

18. Verfahren zum Anmischen von Knochenzement nach einem der vorstehenden Ansprüche 15-17, weiter umfassend:
Abtrennen nach dem Anmischen des Knochenzements des Monomerbehälters vom Mischbehälter.

19. Verfahren zum Anmischen von Knochenzement nach dem vorstehenden Anspruch, weiter umfassend: Einlegen des Mischbehälters nach dem Anmischen als Kartusche in eine Pistole und Entnehmen mit Hilfe eines Kolbens des Knochenzements.

## Claims

1. A vacuum mixing device (1) for bone cement, comprising a mixing container (2), which can be connected to a vacuum source, and a monomer container (10) including a vial (17) containing a monomer, the monomer container (10) being coupleable to the mixing container, wherein at least 20% of the volume of the vial (17) is filled with a gas when a vacuum is not present in the mixing container such that the monomer flows from the monomer container into the mixing container when a vacuum is present in the mixing container (2), and wherein the vacuum mixing device (1) is closed such that ambient air cannot penetrate the vial (17) or the mixing container (2),
**characterized in that** the vacuum mixing device includes a safety bolt (12) that upon removal releases the monomer container (10) toward a coupled state with the mixing container (2).

2. The vacuum mixing device (1) of claim 1, wherein the gas is air.

3. The vacuum mixing device (1) according to the preceding claim, wherein at least 40% of the volume of the vial is filled with the gas.

4. The vacuum mixing device (1) according to any one of the preceding claims, wherein the vial (17) has a membrane (18), and the vacuum mixing device comprises a piercing cannula (19) for piercing the membrane via which the monomer flows into the mixing container (2).

5. The vacuum mixing device (1) according to the preceding claim, wherein the monomer container (10) is capable of being locked when the membrane of the vial is pierced by the piercing cannula.

6. The vacuum mixing device (1) according to the preceding claim, wherein the monomer container (10) includes an upper housing part (15) and a lower housing part (16), the upper housing part (15) and the lower housing part (16) holding the vial (17) and being capable of telescoping relative to each other such that the vial (17) is pushed onto the cannula (19).

7. The vacuum mixing device (1) according to the preceding claim, wherein the upper housing part (15) comprises hooks (22) and the lower housing part (16) comprises lugs (23), wherein upon telescoping of the upper housing part (15) and the lower housing part (16), the hooks (22) are capable of being engaged with the lugs (23) such that upper housing part (15) and the lower housing part (16) may not be returned to their original positions prior to the telescoping of the upper housing part (15) and the lower housing part (16) relative to each other.

8. The vacuum mixing device (1) according to any of claims 5 to 7, wherein the mixing container (2) has a predefined fill quantity determined by a height of the cannula (19) protruding into the monomer container (10).

9. The vacuum mixing device (1) according to any one of the preceding claims, wherein the monomer container (10) is arranged in a handle (13), which can be used to actuate a mixing blade in the mixing chamber via a rod (6), wherein the rod (6) is guided in a plate, which is used as a plunger for expelling the bone cement after the bone cement has been prepared.

10. The vacuum mixing device (1) according to the preceding claim, wherein the monomer container (10) can be coupled to the mixing container via a channel (20) in the handle (13).

11. The vacuum mixing device (1) according to the preceding claim, wherein the channel (20) is disposed in a rod (6) for driving the mixing blade.

12. The vacuum mixing device (1) according to the preceding claim, wherein the rod (6) comprises a predetermined breaking point.

13. The vacuum mixing device (1) according to any one of the preceding claims, wherein, removal of the safety bolt releases the monomer container (10) such that the vial can be pushed onto the cannula (19).

14. The vacuum mixing device (1) according to the preceding claim, wherein the vacuum mixing device comprises means for blocking reinsertion of the removed safety bolt (12).

15. A method for mixing bone cement comprising:
- mixing a monomer from a vial with a powder in a mixing container, the vial being coupleable to the mixing container when a vacuum is applied to the mixing container;
- removing a safety bolt to release the vial toward a coupled state with the mixing container; and
- coupling the vial to the mixing container, wherein, when a vacuum is present, the monomer is pushed out of the vial into the mixing container by a gas that expands as a result of the vacuum, at least 20% of the volume of the vial being filled with the gas when a vacuum is not present in the mixing container, and the vacuum mixing device being closed such that ambient air cannot penetrate the vial or the mixing container.

16. The method for mixing bone cement according to the preceding claim, further comprising piercing the vial with a cannula such that the monomer flows into the mixing container.

17. The method for mixing bone cement according to any one of claims 15-16, wherein the monomer container is locked in engagement with the cannula after the cannula has pierced the vial.

18. The method for mixing bone cement according to any one of claims 15-17, further comprising disengaging the monomer container from the mixing container after the mixing of bone cement.

19. The method for mixing bone cement according to the preceding claim, further comprising after the mixing, introducing the mixing container as a cartridge into a gun and discharging the bone cement via a piston.

## Revendications

1. Dispositif de mélange sous vide (1) pour ciment osseux, comprenant un contenant de mélange (2), lequel peut être raccordé à une source de vide, et un contenant de monomère (10), lequel présente un flacon (17) comprenant un monomère, dans lequel le contenant de monomère (10) peut être relié au contenant de mélange, dans lequel le flacon (17) est rempli d'un gaz au moins à 20 % de son volume, lorsqu'aucun vide n'est appliqué dans le contenant de mélange, de sorte que le monomère circule du contenant de monomère dans le contenant de mélange lorsqu'un vide est appliqué sur le contenant de mélange (2), et dans lequel le dispositif de mélange sous vide (1) est fermé, de sorte qu'aucun air ambiant ne peut pénétrer dans le flacon (17) ou le contenant de mélange (2),
**caractérisé en ce que** le dispositif de mélange sous vide comporte un verrou de sécurité (12), au retrait duquel le contenant de monomère (10) est libéré pour se déplacer dans un état relié au contenant de mélange (2).

2. Dispositif de mélange sous vide (1) selon la revendication 1, dans lequel le gaz est de l'air.

3. Dispositif de mélange sous vide (1) selon l'une quelconque des revendications précédentes, dans lequel le flacon est rempli de gaz au moins à 40 % de son volume.

4. Dispositif de mélange sous vide (1) selon l'une quelconque des revendications précédentes, dans lequel le flacon (17) comprend une membrane (18) et le dispositif de mélange sous vide comporte une canule de ponction (19) destinée à perforer la membrane par l'intermédiaire de laquelle le monomère circule dans le contenant de mélange (2).

5. Dispositif de mélange sous vide (1) selon la revendication précédente, dans lequel le contenant de monomère (10) peut être verrouillé lorsque la membrane du flacon est perforée par la canule de ponction.

6. Dispositif de mélange sous vide (1) selon la revendication précédente, dans lequel le contenant de monomère (10) comprend une partie supérieure de boîtier (15) et une partie inférieure de boîtier (16), dans lequel la partie supérieure de boîtier (15) et la partie inférieure de boîtier (16) contiennent le flacon (17) et les parties peuvent s'emboîter de telle sorte que le flacon (17) est poussé sur la canule (19).

7. Dispositif de mélange sous vide (1) selon la revendication précédente, dans lequel la partie supérieure de boîtier (15) comprend des crochets d'encliquetage (22) et la partie inférieure de boîtier comprend des ergots d'encliquetage (23), dans lequel les crochets d'encliquetage, lorsque les parties de boîtier s'emboîtent, peuvent être amenés en prise avec les ergots d'encliquetage, de sorte que la partie supérieure de boîtier (15) et la partie inférieure de boîtier (16) ne peuvent pas être ramenées dans leur position initiale précédant l'emboîtement mutuel des deux parties de boîtier.

8. Dispositif de mélange sous vide (1) selon l'une quelconque des revendications 5 à 7, dans lequel le contenant de mélange (2) présente une capacité de remplissage prédéfinie, laquelle est définie par une hauteur de la canule (19) qui fait saillie dans le contenant de monomère (10).

9. Dispositif de mélange sous vide (1) selon l'une quelconque des revendications précédentes, dans lequel le contenant de monomère (10) est agencé dans une poignée (13) au moyen de laquelle une pale de mélange peut être actionnée dans le contenant de mélange par l'intermédiaire d'une tige (6), dans lequel la tige (6) est guidée dans une plaque, laquelle sert, après le mélange, de piston pour l'éjection du ciment osseux.

10. Dispositif de mélange sous vide (1) selon la revendication précédente, dans lequel le contenant de monomère (10) peut être relié au contenant de mélange par un canal (20) dans la poignée (13).

11. Dispositif de mélange sous vide (1) selon la revendication précédente, dans lequel le canal (20) est agencé dans une tige (6) destinée à entraîner la pale de mélange.

12. Dispositif de mélange sous vide (1) selon la revendication précédente, dans lequel la tige (6) présente un point de rupture.

13. Dispositif de mélange sous vide (1) selon l'une quelconque des revendications précédentes, dans lequel, une fois le verrou de sécurité retiré, le contenant de monomère (10) est libéré de telle sorte que le flacon peut être poussé sur la canule.

14. Dispositif de mélange sous vide (1) selon la revendication précédente, dans lequel le dispositif de mélange sous vide comprend des moyens afin de bloquer une réinsertion du verrou de sécurité (12) retiré.

15. Procédé pour le mélange d'un ciment osseux, comprenant :
- le mélange d'un monomère provenant d'un flacon dans un contenant de mélange à une poudre, dans lequel le flacon peut être relié au contenant de mélange lorsqu'un vide est appliqué sur le contenant de mélange ;
- le retrait d'un verrou de sécurité, afin de libérer le flacon pour qu'il se déplace dans un état relié au contenant de mélange (2) ; et
- la liaison du flacon au contenant de mélange, dans lequel le monomère, lorsqu'un vide est appliqué, est pressé par un gaz se dilatant en raison du vide hors du flacon dans le contenant de mélange, dans lequel au moins 20 % du volume du flacon est rempli du gaz lorsqu'aucun vide n'est appliqué dans le contenant de mélange, et dans lequel le dispositif de mélange sous vide est fermé de telle sorte qu'aucun air ambiant ne peut pénétrer dans le flacon ou le contenant de monomère.

16. Procédé pour le mélange de ciment osseux selon la revendication précédente, comprenant en outre : la perforation du flacon au moyen d'une canule, afin que le monomère circule dans le contenant de mélange.

17. Procédé pour le mélange de ciment osseux selon l'une quelconque des revendications 15-16, dans lequel le contenant de monomère est encliqueté dans une position reliée à la canule, après que la canule a perforé le flacon.

18. Procédé pour le mélange de ciment osseux selon l'une quelconque des revendications 15-17, comprenant en outre : la séparation, après le mélange du ciment osseux, du contenant de monomère du contenant de mélange.

19. Procédé pour le mélange de ciment osseux selon la revendication précédente, comprenant en outre :
l'insertion du contenant de mélange, après le mélange, en tant que cartouche dans un pistolet et le retrait à l'aide d'un piston du ciment osseux.
